# EUROPEAN PATENT SPECIFICATION

(11) **EP 3 246 337 B1**
(45) Date of publication and mention of the grant of the patent: **21.07.2021**
(21) Application number: 17174064.0
(22) Date of filing: 23.12.2010
(51) Int. Cl.: C07K 14/415, C12N 15/82, A01H 5/00

(54) **TRANSGENIC PLANT HAVING THE PROPERTY OF AN ENHANCED SEED YIELD AND INCREASED GROWTH**
TRANSGENE PFLANZE MIT DER EIGENSCHAFT EINES VERBESSERTEN SAMENERTRAGS UND VERBESSERTEN WACHSTUMS
PLANTE TRANSGÉNIQUE AVEC LA PROPRIÉTÉ D'AUGMENTATION DU RENDEMENT EN GRAINES ET DE PROMOTION DE LA CROISSANCE

(30) Priority: 27.03.2010 DE 102010013166
(43) Date of publication of application: 22.11.2017
(62) Divisional of application: 10805213.5
(73) Proprietor: Technische Universität Kaiserslautern, 67663 Kaiserslautern (DE)
(72) Inventor: NEUHAUS, Ekkehard, 67659 Kaiserslautern (DE); TRENTMANN, Oliver, 67663 Kaiserslautern (DE); WORMIT, Alexandra, London, Greater London SW11 3SL (GB); WINGENTER, Karina, 55595 Spabrücken (DE)
(74) Representative: Patentanwälte Dr. Keller, Schwertfeger Partnerschaft mbB

(56) References cited:
- US-A1- 2007 214 517
- JUNG-IL CHO ET AL: "Expression analysis and functional characterization of the monosaccharide transporters, OsTMTs, involving vacuolar sugar transport in rice (Oryza sativa)", NEW PHYTOLOGIST, vol. 186, no. 3, 23 February 2010 (2010-02-23), pages 657-668, XP055337669, ISSN: 0028-646X, DOI: 10.1111/j.1469-8137.2010.03194.x
- STEFAN WIC: "Charakterisierung vaskuolärer Transportproteine aus Pflanzen unter besonderer Berücksichtigung abiotischer Stressbedingungen", DISSERTATION TECHNISCHE UNIVERSITÄT KAISERSLAUTERN, 1 October 2009 (2009-10-01), pages 1-101, XP002628854, Kaiserslautern
- JOHNSON DEBORAH A ET AL: "The monosaccharide transporter gene family in land plants is ancient and shows differential subfamily expression and expansion across lineages", BMC EVOLUTIONARY BIOLOGY, vol. 6, 21 August 2006 (2006-08-21), pages 64-84, XP021014293, ISSN: 1471-2148 & DATABASE UniProt [Online] 28 November 2006 (2006-11-28), "RecName: Full=Monosaccharide-sensing protein 1; AltName: Full=Monosaccharide transporter 1; AltName: Full=Sugar transporter MSSP1; AltName: Full=Sugar transporter MT1;", retrieved from EBI accession no. UNIPROT:Q96290 Database accession no. Q96290
- WORMIT ALEXANDRA ET AL: "Molecular identification and physiological characterization of a novel monosaccharide transporter from Arabidopsis involved in vacuolar sugar transport", PLANT CELL, vol. 18, no. 12, December 2006 (2006-12), pages 3476-3490, XP009145904, ISSN: 1040-4651
- WINGENTER KARINA ET AL: "Increased Activity of the Vacuolar Monosaccharide Transporter TMT1 Alters Cellular Sugar Partitioning, Sugar Signaling, and Seed Yield in Arabidopsis", PLANT PHYSIOLOGY (ROCKVILLE), vol. 154, no. 2, 13 August 2010 (2010-08-13), pages 665-677, XP009145890, ISSN: 0032-0889
- BUTTNER ET AL: "The monosaccharide transporter(-like) gene family in Arabidopsis", FEBS LETTERS, ELSEVIER, AMSTERDAM, NL, vol. 581, no. 12, 25 May 2007 (2007-05-25) , pages 2318-2324, XP022078428, ISSN: 0014-5793, DOI: 10.1016/J.FEBSLET.2007.03.016

## Description

### Technical Field:

The present invention relates to the use of a transgenic plant that comprises a nucleotide sequence which codes for a TMT (tonoplast monosaccharide transporter) protein, or a homologue thereof, as well as a regulatory nucleotide sequence, operably linked therewith, for the control of an increased gene expression of the TMT protein in the plant cells of the transgenic plant, wherein said TMT protein, or the homologue thereof, exhibits a sequence identity of at least 70% with an amino acid sequence of *Arabidopsis thaliana* TMT (tonoplast monosaccharide transporter) protein TMT1 according to SEQ ID NO: 1, characterized in that said TMT protein, or the homologue thereof, is overexpressed in said plant cells of the transgenic plant resulting in an enhanced seed yield and increased growth as compared with the wild type, for the production of biomass, oils or proteins produced therefrom.

### Background Art:

Many plants are used as the basis for obtaining oils and proteins and therefore represent important raw material carriers for the foodstuffs industry. In this connection, mention may firstly be made of rape (*Brassica napus*), which is of worldwide agronomic importance and stores large amounts of oil and protein in its seed. In Germany alone, about 6.2 million tonnes of rapeseed were harvested in 2009. Because of its oil storage qualities, this plant species is particularly desirable in agriculture for the production of oils and proteins, as is demonstrated by many attempts to enhance the seed yield and the yield of the products produced by the plant. Oil production from rapeseed lies in third place worldwide after soybean oil and palm oil (Jaworski et al., 1993, Canola seed yield in relation to harvest methods. In Janick, JE Simon, eds, New Crops. Wiley, New York, p. 330-301). The main ingredients of rapeseed are predominantly oil (about 35-45%) and protein (20-22%). The oil is accordingly the main energy store of the mature grains.

A close relative of rape is the model organism *Arabidopsis thaliana,* which is used in genetic research and is an excellent representative of the physiology of higher plants. For this reason, the results obtained in the model plant *Arabidopsis thaliana* can also be transferred to other plant species. Because of the ability of plants to act as oil and protein provider, attempts have already been underway for a relatively long time to enhance the plant yield and the seed yield during cultivation in order ultimately to increase the amount of oil or protein produced during production. If the yields of seed can successfully be enhanced, then an increase in the oil and protein harvest per hectare is accordingly to be expected.

Such an approach is followed in WO 2008/092935 A2. That specification describes a method of enhancing the harvest in plants by modulating the expression of a nucleic acid which codes for the YEP (yield enhancing protein) protein. The YEP is selected from a group of cellular proteins, in particular a nucleosome sampling protein 1-like polypeptide (NAP1-like), a LikeSm polypeptide (Lsm protein), a truncated cyclinH (CycH_{Tr}) polypeptide, a Remorin polypeptide and a DREB protein. Transfection of plant cells with YEP leads to an enhanced growth rate of plants and plant parts such as, for example, seeds. The method is accordingly based on the introduction of foreign genes and the expression thereof in transgenic plants. However, there is no reference to the transport of sugar from the cytosol into the plant vacuole.

In order for developing seeds to be able to store oils and proteins, there must first be communication between the green photosynthetically active leaves and the storage organs; that is to say, the products of photosynthesis are transported from the leaves into the seeds, where they are assembled into lipids and proteins. Photosynthesis is a process in which light energy is converted into biochemical energy and in which sugars, starch, amino acids and organic acids are ultimately produced in the leaf. If the sugars in particular are not removed from the leaf quickly enough, a build-up occurs in the cytosol of the individual photosynthetic cells. This sugar build-up signals to the leaf that the photosynthesis was efficient enough, and it is then down-regulated at genetic level (Rolland et al., 2002, Sugar sensing and signaling in plants, Plant Cell 14: 185-205). This means that the genes necessary for efficient photosynthesis are reduced in their expression by high cytosol sugar concentrations, which leads to a fall in photosynthetic efficiency (Koch, 1996, Carbohydrate-modulated gene expression in plants, Ann Rev Plant Physiol Plant Mol Biol 47: 509-540). This in turn has the result that the maximum achievable photosynthesis is inhibited by high sugar concentrations, here in particular glucose, in the cytosol of the photosynthetically active cells.

In various monocotyledonous and dicotyledonous plants such as *Nedicago* (identification no. AC131026), *Vitis vinifera* (identification no. AAX47312) and rice (*Oryza sativa*; identification no. Os02g13560), a protein has been discovered which is responsible for transporting sugar from the cytoplasm of a plant cell into the vacuole thereof. Plant vacuoles play a central role in the long- or short-term storage of sugars. Storage tissues such as beetroot (*Beta vulgaris*) and *Saccharum officinarum* accumulate large amounts of sucrose in order to serve as energy source for plant metabolism. The sugars accumulate in leaves during the day and are released from the vacuole at night.

Finally, a gene has been identified in the plant *Arabidopsis* whose protein product is a sugar transporter which is localised in the vacuole membrane of photosynthetically active cells and is able to import glucose (monosaccharide) from the cytosol into the vacuole (Wormit et al., 2006, Molecular identification and physiological characterization of a novel monosaccharide transporter from Arabidopsis involved in vacuolar sugar transport. Plant Cell 18: 3476-3490). This transport protein, known as tonoplast monosaccharide transporter (TMT), is localised in the membrane of the largest cell organelle. The vacuole as an organelle of a plant cell occupies a volume of about 90% in a photosynthetically active plant cell (Martinola et al., 2007, Vacuolar transporters and their essential role in plant metabolism, J Exp Bot 58: 83-102) and is therefore of immense importance for the storage of sugar on account of its size alone (Neuhaus HE, 2007, Transport of primary metabolites across the plant vacuolar membrane. FEBS Lett 581: 2223-2226). The tonoplast monosaccharide transporter (TMT) protein comprises three isoforms in *Arabidopsis thaliana,* which are denoted TMT1, TMT2 and TMT3. The genes for TMT1 and TMT2 exhibit a tissue- and cell-type-specific expression pattern, whereas the TMT3 gene is expressed only very weakly. By means of gene knockouts it has been possible to show that the plants so changed accumulated markedly less glucose and fructose in the vacuole as compared with wild-type plants. No difference could be detected for the disaccharide sucrose.

In the plant species *Arabidopsis thaliana* alone, more than 60 isoforms of monosaccharide transport proteins have been identified, and these have been classified into various groups (Lalonde et al., 2004, Transport mechanisms for organic forms of carbon and nitrogen between source and sink, Annu, Rev. Plant Biol, 55, 341-372). A number of these transport proteins are likewise localised in the vacuole membrane.

US 2006/0150283 A1 and US 2007/0214517 A1 describe DNA molecules to facilitate exploitation of plants as factories for the synthesis of valuable molecules. The characterisation of TMT (Tonoplast Monosaccharide Transporter) protein was also part of the dissertation which investigated the knock-out of TMT chains resulting in an increased synthesis of sucrose and respiration rate under cold conditions. It was found that AtERD6.5 and BvIMP glucose were transported from the vacuole, whereas AtERD6.5 transported fructose from the vacuole (Charakterisierung von vakuolären Transportproteinen aus Pflanzen unter besonderer Berücksichtigung abiotischer Stressbedingungen, Dissertation Stefan Wic, Technische Universität Kaiserslautern, Oktober 2009). The TMT (Tonoplast Monosaccharide Transporter) gene family was also investigated in more detail and homologues were identified in other plant organisms (Johnson et al., The monosaccharide transporter gene family in land plants is ancient and shows differential subfamily expression and expansion across lineages, 2006. BMC Evol. Biol., 6:64-83; Wingenter et al., Increased Activity of the Vacuolar Monosaccharide Transporter TMT1 Alters Cellular Sugar Partitioning, Sugar Signalling, and Seed Yield in Arabidopsis, 2010. Plant Physiol. 154:665-677). Oryza sativa tonoplast monosaccharide transporters OsTMT1 and OsTMT2 have been found to localise at the tonoplast, and are highly expressed in bundle sheath cells and in vascular parenchyma and companion cells in leaves (Jung-II Cho," Expression analysis and functional characterization of the monosaccharide transporters, OsTMTs, involving vacuolar sugar transport in rice (Oryza sativa), New Phytologist 186:657-668 (2010)). Furthermore, glucose uptake studies using vacuoles isolated from transgenic mutant Arabidopsis (tmt1-2-3) expressing OsTMT1 demonstrated that OsTMTs are capable of transporting glucose into vacuoles.

### Disclosure of Invention:

Against this background, it is an object of the present invention to provide a transgenic plant which seed yield and growth can be increased
That object is achieved by the use of a transgenic plant having the features of claim 1. Preferred embodiments are to be found in the dependent claims.

The inventors have found, surprisingly, that overexpression mutants of the tonoplast monosaccharide transporter (TMT) protein lead to a concentration of sugars such as glucose in the vacuole of plant cells, while the sugar concentrations in the cell cytosol were lower.

Overexpression of the sugar transport protein resulted in an enhanced expression of the genes responsible for photosynthesis (also observable by the rise in the photosynthetic efficiency) and ultimately in an increase in the seed weights of the plant. The number of seeds per silique remained unchanged. The number of total seed per plant increased significantly, however. This leads to the conclusion that the overexpression of a sugar transport protein localised in the vacuole membrane results in an increase in the seed yield and in the promotion of growth in plants. Because the transport proteins for transporting mono- and di-saccharides from the cytoplasm into the vacuole are to be found in all vacuole-containing plant cells, the method according to the invention can be used in both monocotyledonous and dicotyledonous plants. If the results obtained in this invention are transferred from *Arabidopsis* to rape, then the method according to the invention can be used to enhance the oil and protein yield in rape significantly. By means of the invention it is thereby possible to increase the harvests of cultivated and useful plants that are of worldwide importance, and products produced therefrom.

The method according to the invention is based on the overexpression of the tonoplast monosaccharide transporter (TMT) protein, or a homologue or analogue thereof, in isogenic or transgenic cells of monocotyledonous or dicotyledonous plants. The cDNA clone according to the invention (identification no. 8B8T74) codes for 734 amino acids and exhibits a sequence similarity of 32% with the bacterial glucose transporter GTR from Synechocystis and 26% similarity with the *Arabidopsis* glucose transport protein STP1 localised in the plasma membrane (Wormit et al., 2006, Molecular identification and physiological characterization of a novel monosaccharide transporter from Arabidopsis involved in vacuolar sugar transport. Plant Cell 18: 3476-3490). This protein is known as tonoplast monosaccharide transporter protein (TMT)1. By means of polymerase chain reaction it was possible to amplify two additional TMT isoforms in addition to TMT1, the TMT2 protein containing 739 amino acid residues and the TMT3 protein containing 729 amino acid residues.

The TMT1 protein has 12 transmembrane domains and a relatively large centrally localised hydrophilic loop which connects domains 6 and 7 to one another. This loop contains about 320 amino acid residues and is accordingly four to five times larger than corresponding structures in other known monosaccharide transport proteins in prokaryotes and eukaryotes. The cDNA sequence of the tonoplast monosaccharide transporter (TMT) protein in *Arabidopsis thaliana* is shown in SEQ ID NO: 2 and the amino acid sequence derived therefrom is shown in SEQ ID NO: 1.

The tests carried out in the following examples clearly show that, by overexpression of the tonoplast monosaccharide transporter (TMT) protein TMT1 of *Arabidopsis thaliana,* the seed weights could be increased as compared with wild-type plants and the oil and protein contents in mature *Arabidopsis* seeds increased. Furthermore, the crop yield of the seeds in various *Arabidopsis* lines could also be increased. The overexpression further led to *Arabidopsis* plants developing markedly more quickly as compared with the wild type and exhibiting increased growth. This is presumably attributable to the fact that the overexpression of TMT protein leads to an accumulation of the sugar from the cytosol in the vacuole of the plant cell and thus increases the rate of photosynthesis. Alternatively, it can be assumed that the larger seeds of the overexpression mutants provide the seedlings with more energy, which would represent an advantage over the wild-type seedlings in particular in the early phase of development. Because a number of sugar transport proteins that are localised in the vacuole membrane are known in both monocotyledonous and dicotyledonous plant cells, it is understandable that the principle according to the invention is generally transferable to corresponding sugar transport proteins.

Because the TMT (tonoplast monosaccharide transporter) protein identified in *Arabidopsis thaliana* also exhibits sequence homologies to transport proteins in other plant cells (see introduction), homologous transport proteins to the TMT1 transport protein of *Arabidopsis thaliana* are also included in the invention.

A "homologue" within the scope of the invention therefore denotes a transport protein which exhibits a high sequence identity with the TMT (tonoplast monosaccharide transporter) protein TMT1 of *Arabidopsis thaliana.* Preferably, a homologue has a sequence identity of 50%, 60%, 70%, 75%, 80%, 85%, 90%, 95%, 96%, 97%, 98%, 99% or 100% with an amino acid sequence of *Arabidopsis thaliana* TMT (tonoplast monosaccharide transporter) protein TMT1.

By way of distinction, an "analogue" within the scope of the invention denotes a sugar transport protein which is localised in the vacuole membrane and performs the function of transporting sugar from the cytoplasm into the vacuole without there necessarily having to be a sequence homology to the TMT (tonoplast monosaccharide transporter) protein. An analogue therefore has the same function as the TMT1 protein but can be structurally different. Unlike a homologue, a genetic relationship is not a prerequisite.

Preferably, the TMT (tonoplast monosaccharide transporter) protein TMT1 of *Arabidopsis thaliana* having an amino acid sequence according to SEQ ID No 1, or a homologue or analogue thereof, is used to enhance the seed yield to promote the growth of monocotyledonous or dicotyledonous plants. For overexpression or regulated gene expression, a nucleotide sequence which codes for the TMT (tonoplast monosaccharide transporter) protein, or a homologue or analogue thereof, is preferably introduced into the plant cells and overexpressed in the plant cells under the control of a constitutive or inducible promoter. Examples of constitutive promoters are the cauliflower mosaic virus 35S promoter, the *Agrobacterium tumefaciens* nopaline/octopine synthase promoter or the maize Emu promoter. In addition there are light-inducible promoters, such as, for example, the RUBISCO small subunit rbcS promoter (rice, tomato). The methods for gene expression of genes in plant cells are generally conventionally known and described in the literature, as are the specific cloning methods and the controlled regulation of gene expression. In addition to the promoters, termination sequences for the controlled expression of genes may also be necessary. For the selection of seedlings it is additionally possible to introduce marker genes (e.g. GFP) or other selection genes for which specific antibodies for detection are available, for example against the c-myc motif.

Gene expression can take place either by cloning of a plasmid with the corresponding nucleotide sequences or by incorporation into the genome of the plant cell. Stable integration into the genome of the plant cell and the possibility of targeted regulation of gene expression leads to a stable expression of the TMT (tonoplast monosaccharide transporter) protein, or a homologue or analogue thereof, in the plant cell.

It is possible according to the invention that the overexpression of TMT (tonoplast monosaccharide transporter) protein, or a homologue or analogue thereof, is possible in both isogenic and transgenic plant cells. In isogenic plant cells, the endogenous gene for the transporter protein in question is overexpressed and ultimately leads to enhanced seed production and more rapid growth of the plant as compared with the wild type. In addition, it is also possible to use sugar transport protein from other plant species for the overexpression, so that a transgenic gene expression in the transfected plant cells is obtained. For example, the gene for the TMT (tonoplast monosaccharide transporter) protein TMT1 of *Arabidopsis thaliana* can be transfected into the rape plant *Brassica napus* and the gene can be expressed.

The expression of the TMT (tonoplast monosaccharide transporter) protein, or of a homologue or analogue thereof, in plant cells, in particular in plant cells having a high vacuolar storage capacity, such as *Brassica napus,* is particularly interesting economically because larger amounts of oil or protein can be produced here. For example, endosperm production or potato tuber production can be increased by the targeted insertion of the gene according to the invention for TMT (tonoplast monosaccharide transporter) protein and its expression, as a result of which a higher starch yield is achieved, which in turn increases the production of bioethanol.

The targeted incorporation and the expression of TMT (tonoplast monosaccharide transporter) protein, or a homologue or analogue thereof, in isogenic or transgenic plants cells further leads to an enhancement of the yield per unit area, because the plants reach harvest size much earlier and can accordingly be modified. It is therefore possible to achieve more harvests on the same cultivation area in a shorter time. The method according to the invention leads to marked increases in harvest with a markedly improved biomass yield per unit area as compared with the wild-type plants.

The method according to the invention can in principle be used in all monocotyledonous and dicotyledonous plants. Cultivated plants and useful plants in particular are economically interesting, such as, for example, the rape *Brassica napus.* Preference is given, for example, to the species and their higher genera of *Arabidopsis thaliana, Brassica napus, Brassica oleracea, Brassica rapa, Arachis hypogea, Boechera stricta, Bruguiera gymnorhiza, Citrus paradisi, Poncirus trifoliate, Euphorbia esula, Fragaria vesca, Gossypium hirsutum, Gossypium raimondii, Glycine max, Helianthus annuus, Ipomoea nil, Lycopersicon esculentum, Lactuca perennis, Lactuca saligna, Lactuca serriola, Lactuca sativa, Lotus japonicus, Malus domestica, Medicago truncatula, Nicotiana tabacum, Oryza australiensis, Oryza brachyanth, Oryza punctata, Oryza ridleyi, Oryza rufipogon, Oryza sativa, Populus trichocarpa, Poncirus trifoliata, Prunus persica, Solanum tuberosum Sorghum bicolor, Triticum aestivum, Zea mays, Saccharum officinarum, Panicum virgatum, Miscanthus, Vitis vinifera, Cannabis sativa.*

The invention relates further to a transgenic plant having the property of an enhanced seed yield and increased growth as compared with the wild type, comprising a nucleotide sequence which codes for a TMT (tonoplast monosaccharide transporter) protein, as well as a regulatory nucleotide sequence, operably linked therewith, for the control of an increased gene expression of the TMT protein in the plant cells of the transgenic plant. Preferably, the nucleotide sequence codes for TMT (tonoplast monosaccharide transporter) protein TMT1 of *Arabidopsis thaliana* according to SEQ ID NO: 1, or a homologue or analogue thereof.

The transgenic plant according to the invention can be used in the cultivation of cultivated plants or useful plants. Further, it can be used in the production of biomass, oils or proteins produced therefrom.

The invention also includes the gene construct which comprises a nucleotide sequence which codes for a TMT (tonoplast monosaccharide transporter) protein TMT1 of *Arabidopsis thaliana* according to SEQ ID NO: 1, or a homologue or analogue thereof, as well as regulatory nucleotide sequences, operably linked therewith, for the control of an increased gene expression of the TMT protein in a plant cell. The invention is explained in greater detail in the following figures.

### Brief Description of Drawings:

In the figures
- Fig. 1: shows a cloning diagram for the production of an overexpression construct of TMT (tonoplast monosaccharide transporter) protein;
- Fig. 2: shows a schematic representation of the T-DNA insertions of the double mutant *TMT1-2;*
- Fig. 3: shows the thousand-kernel weight of *Arabidopsis* seeds;
- Fig. 4: shows oil and lipid contents of *Arabidopsis* seeds;
- Fig. 5: shows the number of seeds per silique in *Arabidopsis;*
- Fig. 6: shows the total weight of the seeds per *Arabidopsis* plant;
- Fig. 7: shows the development of *Arabidopsis* plants after 15 and 34 days.

### Best mode for carrying out the invention:

### Examples:

### Cloning of the plasmid construct for the overexpression of TMT protein (Attmt1)

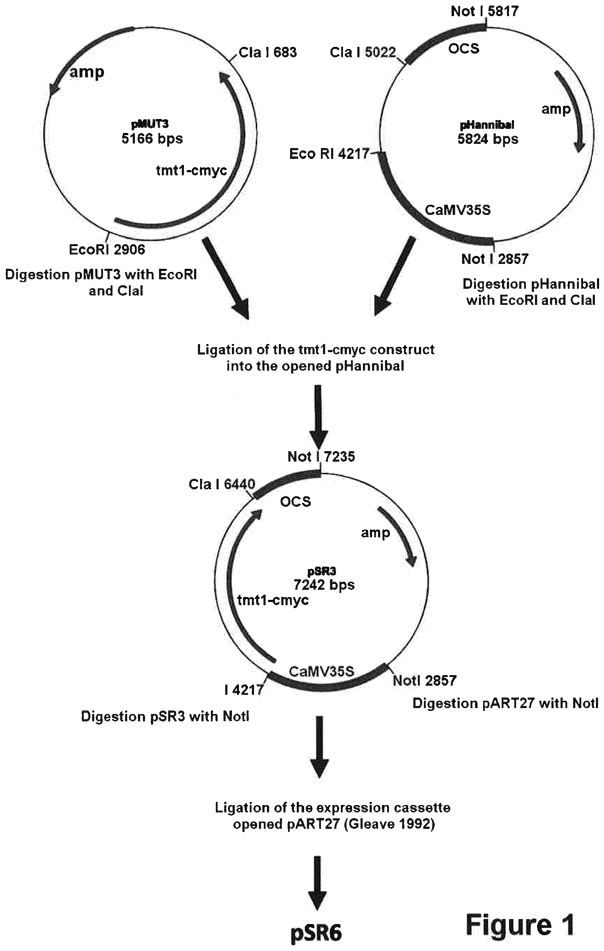

For the expression of the sugar transport protein, the cDNA of TMT (tonoplast monosaccharide transporter) protein TMT1 of *Arabidopsis thaliana* was used (= *Attmt-*cDNA). For the production of the overexpression construct, a changed Attmt-cDNA, which contains a c-myc motif in the region of the hydrophilic loop between the putative transmembrane domains 6 and 7, was used. This is the construct pMUT3 (see Fig. 1).

The entire cDNA sequence was first cut from the plasmid pMUT3 with the restriction enzymes EcoRI and Clal and ligated into the plasmid pHannibal linearised with the same enzymes (pSR3). There formed an expression cassette in which a cauliflower mosaic virus 35S promoter was located upstream of the TMT1-cDNA and an OCS terminator was located downstream of the gene at the downstream end (polyadenylation signal of the octopine synthase gene). A further restriction digestion with the restriction enzyme Notl from plasmid pSR3 led to isolation of the cassette, which was finally introduced into the plant transformation vector pART27 (pSR6).

The cDNA sequence of *Attmt1* (SEQ ID NO: 2) with an integrated c-myc motif (emphasised in red) is as follows:

The cloning diagram shown in Fig. 1 shows the production of the overexpression construct pSR6 based on the *Attmt1* cDNA.

The sequences of the vectors pMUT3 and pSR3 so obtained are given in the sequence listing or SEQ ID NO. 3 and SEQ ID NO. 4.

### Production of Attmt1 overexpression lines

For the introduction of the overexpression construct into the genome of *Arabidopsis* plants, the so-called "floral dip process" was used. In this process, inflorescences were dipped in a suspension of agrobacteria (strain GV3101) which had previously been transformed with the construct pSR6. The transformed plants were homozygotic t-DNA insertion cell lines, in which both the native *tmt1* gene and the native *tmt2* gene had been deleted by insertion of a transfer DNA (see Wormit et al., 2006: Molecular identification and physiological characterization of a novel monosaccharide transporter from Arabidopsis involved in vacuolar sugar transport. Plant Cell 18: 3476-3490). In this manner, the production of the native mRNA for *tmt1* and *tmt2* was suppressed and co-suppression of the artificial mRNA was prevented. Instead of a deletion of the native *tmt1* and *tmt2* genes, a heterologous *tmt* sequence can also be overexpressed. The expression of a native tmt1-cDNA sequence is further possible.

When seed formation was complete, the plants were harvested and made to germinate on kanamycin-containing agar plates for the selection of transgenic cell lines. Because the transfected plant cells carry a kanamycin resistance gene, only those seedlings in which transfection with the *tmt1*-cDNA has been successful can develop, because such cells develop resistance to the antibiotic.

The selection of the overexpression cell lines was carried out by a Northern blot analysis, by selecting those lines which exhibit a marked increase in the *Attmt1* transcript amount as compared with the wild type.

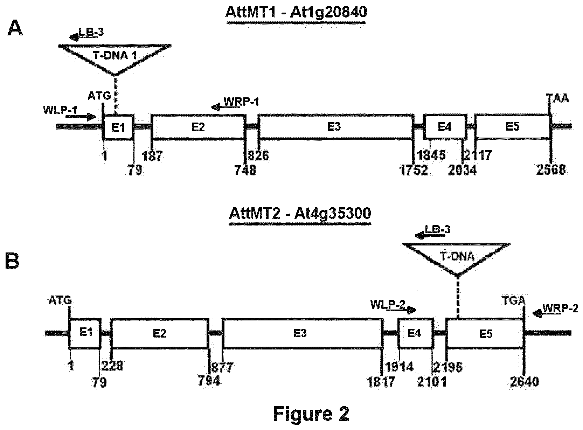

This situation is illustrated again schematically in Fig. 2. The individual t-DNA insertions into the Exon regions of the *Attmt1* and *Attmt2* cDNA are shown.

### Cultivation of plants for seed analysis

Before germination, *Arabidopsis* seeds were incubated for two days in the dark at 4°C for inhibition. Wild-type plants, the homozygotic mutant cell line *tmt1-2* and the three *tmt1* overexpression cell lines 1, 4 and 10 were cultivated on plates for nine weeks and cultivated at 22°C under short-day conditions (10 hours' light, 14 hours' darkness) in a growth chamber. Thereafter, the plants were switched to long-day conditions (14 hours' light, 10 hours' darkness) at 22°C. Watering was continued for three weeks under those conditions. Then watering was stopped and the plants were cultivated further until complete dryness of the infructescence was noted. The seeds of the individual plants were carried out with the aid of a seed collector (Aracon 720).

### Seed analysis

For fatty acid quantification, 0.1 g of fully mature and air-dried seeds was homogenised in a mortar in liquid nitrogen. Then a volume of 1.5 ml of isopropanol was added and further homogenisation was carried out. The suspension was transferred to a reaction vessel having a volume of 1.5 ml and incubated for 12 hours at 4°C in a laboratory agitator at 100 rpm.

Samples were then centrifuged for 10 min. at 12,000 g and the supernatant was transferred to previously measured 1.5 ml reaction vessels. The reaction vessels were incubated for 8 hours at 60°C in order to evaporate the isopropanol. The total lipid content was then determined by gravimetry.

For protein quantification of the seed, 0.1 g of seed was homogenised in a mortar at room temperature. Then a volume of 1000 µl of buffer medium (50 mM HEPES, 5 mM MGCl₂, pH 7.5, 1% Triton X100, 15% glycerol, 2% SDS, 1 mM EDTA, PMSF, 1/100 (v/v)) was added and further homogenisation was carried out. The suspension was transferred to 1.5 ml reaction vessels, and the samples were centrifuged for 10 min. at 12,000 g at room temperature. The supernatant was transferred to new 1.5 ml reaction vessels and the proteins were quantified with BCA reagent according to the manufacturer's recommendations.

### Results

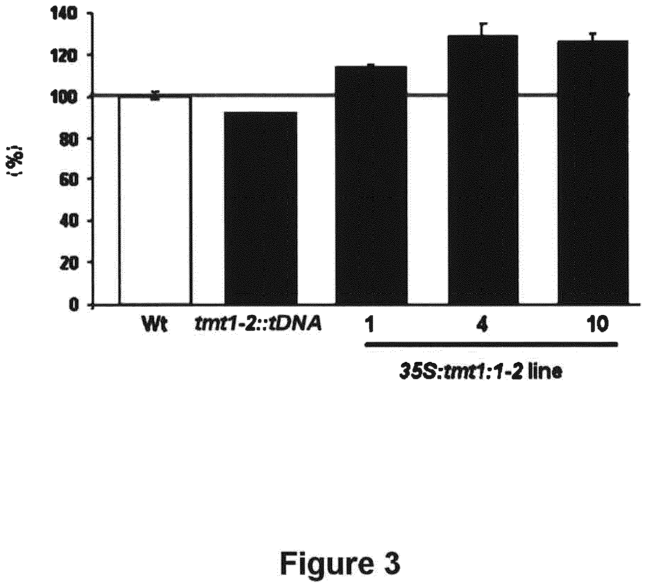

Fig. 3 shows the thousand-kernel weight of *Arabidopsis* seeds. As control there was used a wild-type *Arabidopsis* plant as well as a *tmt1-2* mutant which exhibited greatly reduced vacuolar TMT activity (Wormit et al., 2006, Molecular identification and physiological characterization of a novel monosaccharide transporter from Arabidopsis involved in vacuolar sugar transport. Plant Cell 18: 3476-3490). Overexpression with overexpression lines 1, 4 and 10 leads to a significant increase in the thousand-kernel weight as compared with the wild type.

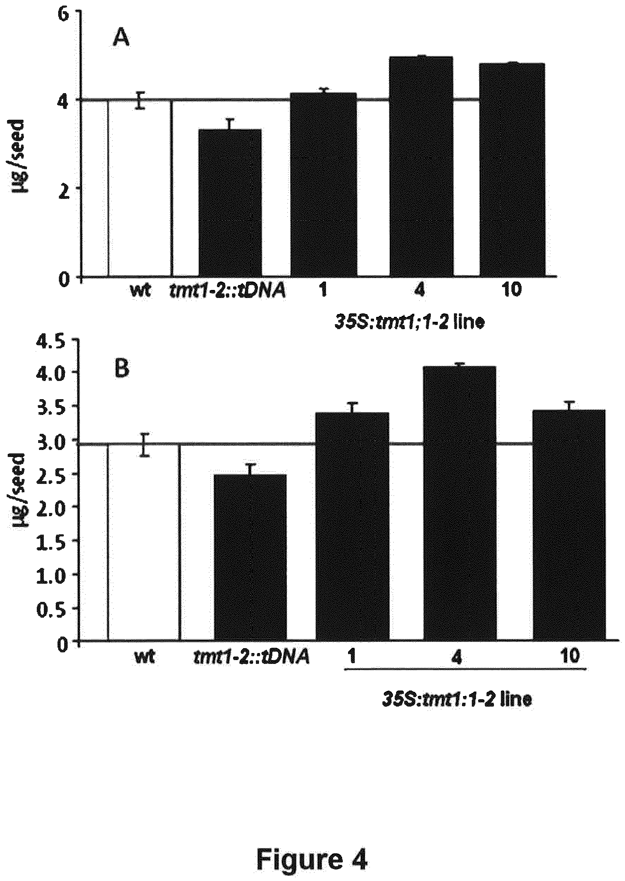

Fig. 4 shows the oil and lipid contents of *Arabidopsis* seeds. The oil content (Fig. 4A) and the protein content (Fig. 4B) are increased as compared with the wild-type *Arabidopsis* plants (WT) and the three independent TMT1 overexpression lines.

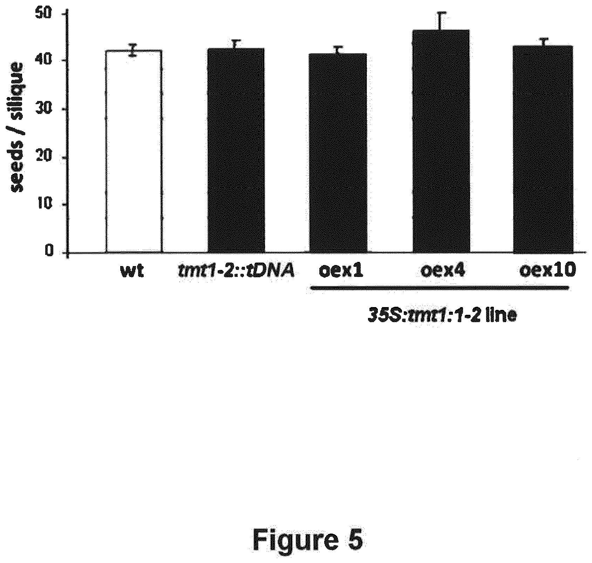

Fig. 5 shows the number of seeds per silique in *Arabidopsis.* The average number of seeds per silique of wild-type *Arabidopsis* plants (WT) and the three independent TMT1 overexpression lines is virtually the same.

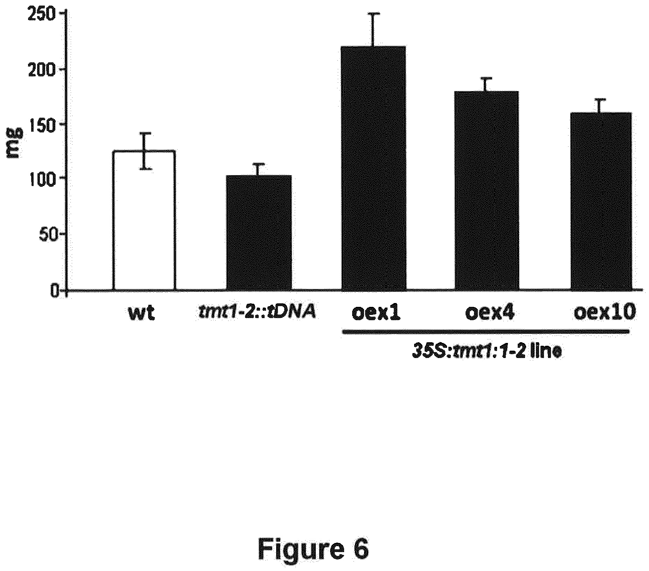

Fig. 6 summarises the total weight of the seeds per *Arabidopsis* plant. The average total weight of all mature seeds on wild-type *Arabidopsis* plants (WT) and the three independent TMT1 overexpression lines is shown. The total weight of the seeds is markedly increased in the overexpression lines as compared with the wild type. In Fig. 6A, this can already be seen visually, while in Fig. 6B a quantification of the seeds per plant was carried out.

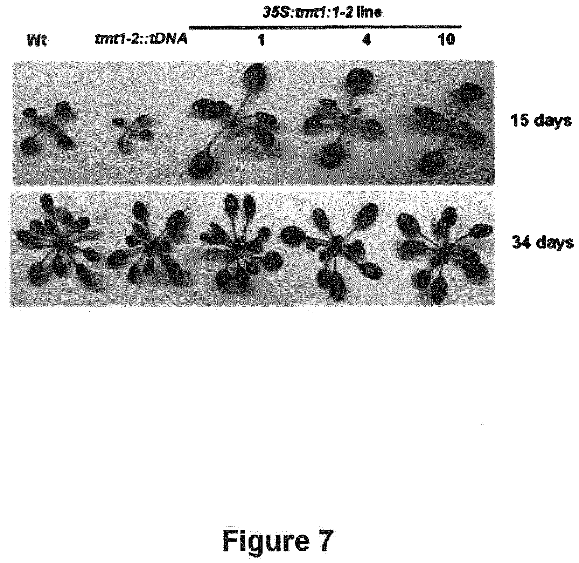

Fig. 7 shows the development of *Arabidopsis* plants after 15 and 34 days. The overexpression lines exhibit markedly stimulated growth after only 15 days and are markedly larger compared with the wild-type *Arabidopsis* plant. An overexpression of TMT1 therefore leads to increased growth of the plants.

In summary, the results clearly show that the seed yield, the germination capacity and the growth of plants can be increased by an overexpression of the TMT (tonoplast monosaccharide transporter) protein.

### Text description relating to the figures:

- Fig. 1:: Cloning diagram for production of the *Attmt1* overexpression construct pSR6 amp: ampicillin resistance gene; CaMV-35S: cauliflower mosaic virus 35S promoter; OCS: polyadenylation signal from the octopine synthase gene; tmt1-cmyc: cDNA of Attmt1 with inserted cmyc motif;
- Fig. 2:: Schematic representation of the T-DNA insertions of the double mutant tmt1-2 *AttMT1* with insertion of the T-DNA, B: *AttMT2* with insertion of the T-DNA;
- Fig. 3: Thousand-kernel weight of *Arabidopsis* seeds. The weight of wild-type *Arabidopsis* plants (Wt, based on 100%) and three independent TMT1 overexpression lines is shown. *tmt1-2* is a mutant which does not exhibit vacuolar TMT activity (Wormit *et al.,* 2006). The data are statistically significant (average values +/- standard error) and are from three independent plant cultivations;
- Fig. 4: Oil and lipid contents of *Arabidopsis* seeds. The oil content (A) and the protein content (B) of wild-type *Arabidopsis* plants (Wt) and three independent TMT1 overexpression lines are shown. *tmt1-2* is a mutant which does not exhibit vacuolar TMT activity (Wormit *et al.,* 2006). The data are statistically significant (average values +/- standard error) and are from three independent plant cultivations;
- Fig. 5: Number of seeds per silique in *Arabidopsis.* The average number of seeds per silique of wild-type *Arabidopsis* plants (Wt) and three independent TMT1 overexpression lines is shown. *tmt1-2* is a mutant which does not exhibit vacuolar TMT activity (Wormit *et al.,* 2006). The data are statistically significant (average values +/- standard error) and are from three independent plant cultivations;
- Fig. 6: Total weight of the seeds per *Arabidopsis* plant. The average total weight of all mature seeds on wild-type *Arabidopsis* plants (Wt) and three independent TMT1 overexpression lines is shown. *tmt1-2* is a mutant which does not exhibit vacuolar TMT activity (Wormit *et al.,* 2006). A. Visual representation, B. Seed quantification. The data are statistically significant (average values +/- standard error) and are from three independent plant cultivations;
- Fig. 7: Development of *Arabidopsis* plants after 15 and 34 days. The development state of wild-type Arabidopsis plants (Wt), tmt1-2 mutants, which lack the activity of the vacuolar glucose transporter TMT (Wormit *et al.,* 2006), and three independent TMT1 overexpression lines is shown. The more rapid development of the TMT overexpression can clearly be seen.

## Claims

1. Use of a transgenic plant that comprises a nucleotide sequence which codes for a TMT (tonoplast monosaccharide transporter) protein, or a homologue thereof, as well as a regulatory nucleotide sequence, operably linked therewith, for the control of an increased gene expression of the TMT protein in the plant cells of the transgenic plant, wherein said TMT protein, or the homologue thereof, exhibits a sequence identity of at least 70% with an amino acid sequence of *Arabidopsis thaliana* TMT (tonoplast monosaccharide transporter) protein TMT1 according to SEQ ID NO: 1, **characterized in that** said TMT protein, or the homologue thereof, is overexpressed in said plant cells of the transgenic plant resulting in an enhanced seed yield and increased growth as compared with the wild type, for the production of biomass, oils or proteins produced therefrom.

2. The use according to claim 1, **characterised in that** the nucleotide sequence codes for TMT (tonoplast monosaccharide transporter) protein TMT1 of *Arabidopsis thaliana* according to SEQ ID NO: 1.

3. The use according to claim 1, **characterised in that** the TMT (tonoplast monosaccharide transporter) protein, or the homologue thereof, comprises the amino acid sequence of *Arabidopsis thaliana* TMT1 according to SEQ ID NO: 1.

4. The use according to claim 1, 2 or 3, **characterised in that** the TMT (tonoplast monosaccharide transporter) protein, or the homologue thereof, is overexpressed in the plant cells under the control of a constitutive or inducible promoter.

5. The use according to any one of claims 1 to 4, **characterised in that** the TMT (tonoplast monosaccharide transporter) protein exhibits a sequence identity of 70%, 75%, 80%, 85%, 90%, 95%, 96%, 97%, 98%, 99% or 100% with an amino acid sequence of *Arabidopsis thaliana* TMT (tonoplast monosaccharide transporter) protein TMT1 according to SEQ ID NO: 1.

6. The use according to any one of claims 1 to 5, **characterised in that** the plants are cultivated or useful plants of the species or higher genera of *Arabidopsis thaliana, Brassica napus, Brassica oleracea, Brassica rapa, Arachis hypogea, Boechera stricta, Bruguiera gymnorhiza, Citrus paradisi, Poncirus trifoliate, Euphorbia esula, Fragaria vesca, Gossypium hirsutum, Gossypium raimondii, Glycine max, Helianthus annuus, Ipomoea nil, Lycopersicon esculentum, Lactuca perennis, Lactuca saligna, Lactuca serriola, Lactuca sativa, Lotus japonicus, Malus domestica, Medicago truncatula, Nicotiana tabacum, Oryza australiensis, Oryza brachyanth, Oryza punctata, Oryza ridleyi, Oryza rufipogon, Oryza sativa, Populus trichocarpa, Poncirus trifoliata, Prunus persica, Solanum tuberosum Sorghum bicolor, Triticum aestivum, Zea mays, Saccharum officinarum, Panicum virgatum, Miscanthus, Vitis vinifera, Cannabis sativa.*

## Patentansprüche

1. Verwendung einer transgenen Pflanze, umfassend eine Nukleotidsequenz, welche für ein TMT (Tonoplast-Monosaccharid-Transporter)-Protein oder ein Homolog davon kodiert, sowie eine damit operabel verbundene regulatorische Nukleotidsequenz zur Steuerung einer erhöhten Genexpression des TMT-Proteins in den Pflanzenzellen der transgenen Pflanze, wobei das TMT-Protein oder das Homolog davon eine Sequenzidentität von wenigstens 70% mit einer Aminosäuresequenz des *Arabidopsis thaliana* TMT (Tonoplast-Monosaccharid-Transporter)-Proteins TMT1 gemäß SEQ ID NO: 1 aufweist, **dadurch gekennzeichnet, dass** das TMT-Protein oder das Homolog davon in den Pflanzenzellen der transgenen Pflanze überexprimiert wird, was zu einer erhöhten Samenausbeute und einem erhöhten Wachstum im Vergleich zum Wildtyp führt, zur Herstellung von Biomasse, Ölen oder daraus hergestellten Proteinen.

2. Verwendung nach Anspruch 1, **dadurch gekennzeichnet, dass** die Nukleotidsequenz für das TMT (Tonoplast-Monosaccharid-Transporter)-Protein TMT1 von *Arabidopsis thaliana* gemäß SEQ ID NO: 1 kodiert.

3. Verwendung nach Anspruch 1, **dadurch gekennzeichnet, dass** das TMT (Tonoplast-Monosaccharid-Transporter)-Protein oder das Homolog davon die Aminosäuresequenz von *Arabidopsis thaliana* TMT1 gemäß SEQ ID NO: 1 umfasst.

4. Verwendung nach Anspruch 1, 2 oder 3, **dadurch gekennzeichnet, dass** das TMT-Protein (Tonoplast-Monosaccharid-Transporter) oder das Homolog davon in den Pflanzenzellen unter der Kontrolle eines konstitutiven oder induzierbaren Promotors überexprimiert wird.

5. Verwendung nach einem der Ansprüche 1 bis 4, **dadurch gekennzeichnet, dass** das TMT (Tonoplast-Monosaccharid-Transporter)-Protein eine Sequenzidentität von 70 %, 75 %, 80 %, 85 %, 90 %, 95 %, 96 %, 97 %, 98 %, 99 % oder 100 % mit einer Aminosäuresequenz des TMT (Tonoplast-Monosaccharid-Transporter)-Proteins TMT1 von *Arabidopsis thaliana* gemäß SEQ ID NO: 1 aufweist.

6. Verwendung nach einem der Ansprüche 1 bis 5, **dadurch gekennzeichnet, dass** die Pflanzen Kultur- oder Nutzpflanzen der Arten oder höheren Gattungen *Arabidopsis thaliana, Brassica napus, Brassica oleracea, Brassica rapa, Arachis hypogea, Boechera stricta, Bruguiera gymnorhiza, Citrus paradisi, Poncirus trifoliate, Euphorbia esula, Fragaria vesca, Gossypium hirsutum, Gossypium raimondii, Glycine max, Helianthus annuus, Ipomoea nil, Lycopersicon esculentum, Lactuca perennis, Lactuca saligna, Lactuca serriola, Lactuca sativa, Lotus japonicus, Malus domestica, Medicago truncatula, Nicotiana tabacum, Oryza australiensis, Oryza brachyanth, Oryza punctata, Oryza ridleyi, Oryza rufipogon, Oryza sativa, Populus trichocarpa, Poncirus trifoliata, Prunus persica, Solanum tuberosum Sorghum bicolor, Triticum aestivum, Zea mays, Saccharum officinarum, Panicum virgatum, Miscanthus, Vitis vinifera, Cannabis sativa* sind.

## Revendications

1. Utilisation d'une plante transgénique qui comprend une séquence de nucléotides qui code pour une protéine TMT (transporteur tonoplastique des monosaccharides), ou un homologue afférent, de même qu'une séquence de nucléotides de régulation, qui lui est liée de manière opérationnelle, pour la commande d'une expression génique augmentée de la protéine TMT dans les cellules de plante de la plante transgénique, dans laquelle ladite protéine TMT, ou l'homologue afférent, présente une identité de séquence d'au moins 70 % par rapport à une séquence d'acides aminés d'une protéine TMT (transporteur tonoplastique des monosaccharides) TMT1 de *Arabidopsis thaliana* conformément à SEQ ID NO: 1, **caractérisée en ce que** ladite protéine TMT, ou l'homologue afférent, est surexprimée dans lesdites cellules de plante de la plante transgénique, ce qui conduit à une augmentation du rendement en graines et à une augmentation de la croissance par comparaison avec le type sauvage, pour la production d'une biomasse, d'huiles ou de protéines qui sont produites sur cette base.

2. Utilisation selon la revendication 1, **caractérisée en ce que** la séquence de nucléotides code pour la protéine TMT (transporteur tonoplastique des monosaccharides) TMT1 de *Arabidopsis thaliana* conformément à SEQ ID NO : 1.

3. Utilisation selon la revendication 1, **caractérisée en ce que** la protéine TMT (transporteur tonoplastique des monosaccharides), ou l'homologue afférent, comprend la séquence d'acides aminés de TMT1 de *Arabidopsis thaliana* conformément à SEQ ID NO : 1.

4. Utilisation selon la revendication 1, 2 ou 3, **caractérisée en ce que** la protéine TMT (transporteur tonoplastique des monosaccharides), ou l'homologue afférent, est surexprimée dans les cellules de plante sous la commande d'un promoteur constitutif ou inductible.

5. Utilisation selon l'une quelconque des revendications 1 à 4, **caractérisée en ce que** la protéine TMT (transporteur tonoplastique des monosaccharides) présente une identité de séquence de 70 %, 75 %, 80 %, 85 %, 90 %, 95 %, 96 %, 97 %, 98 %, 99 % ou 100 % par rapport à une séquence d'acides aminés de la protéine TMT (transporteur tonoplastique des monosaccharides) TMT1 de *Arabidopsis thaliana* conformément à SEQ ID NO : 1.

6. Utilisation selon l'une quelconque des revendications 1 à 5, **caractérisée en ce que** les plantes sont cultivées ou sont des plantes utiles des espèces ou des genres supérieurs de *Arabidopsis thaliana, Brassica napus, Brassica oleracea, Brassica rapa, Arachis hypogea, Boechera stricta, Bruguiera gymnorhiza, Citrus paradisi, Poncirus trifoliate, Euphorbia esula, Fragaria vesca, Gossypium hirsutum, Gossypium raimondii, Glycine max, Helianthus annuus, Ipomoea nil, Lycopersicon esculentum, Lactuca perennis, Lactuca saligna, Lactuca serriola, Lactuca sativa, Lotus japonicus, Malus domestica, Medicago truncatula, Nicotiana tabacum, Oryza australiensis, Oryza brachyanth, Oryza punctata, Oryza ridleyi, Oryza rufipogon, Oryza sativa, Populus trichocarpa, Poncirus trifoliata, Prunus persica, Solanum tuberosum Sorghum bicolor, Triticum aestivum, Zea mays, Saccharum officinarum, Panicum virgatum, Miscanthus, Vitis vinifera, Cannabis sativa.*


### SEQUENCE LISTING

<110> Technische Universität Kaiserslautern
<120> Transgenic Plant having the property of an enhanced seed yield and
   increased growth
<130> TMT Transporter
<160> 4
<170> PatentIn version 3.3
<210> 1
   <211> 734
   <212> PRT
   <213> Arabidopsis thaliana
<400> 1
   Met Lys Gly Ala Thr Leu Val Ala Leu Ala Ala Thr Ile Gly Asn Phe
   1               5                   10                  15
   Leu Gln Gly Trp Asp Asn Ala Thr Ile Ala Gly Ala Met Val Tyr Ile
               20                  25                  30
   Asn Lys Asp Leu Asn Leu Pro Thr Ser Val Gln Gly Leu Val Val Ala
           35                  40                  45
   Met Ser Leu Ile Gly Ala Thr Val Ile Thr Thr Cys Ser Gly Pro Ile
       50                  55                  60
   Ser Asp Trp Leu Gly Arg Arg Pro Met Leu Ile Leu Ser Ser Val Met
   65                  70                  75                  80
   Tyr Phe Val Cys Gly Leu Ile Met Leu Trp Ser Pro Asn Val Tyr Val
                   85                  90                  95
   Leu Cys Phe Ala Arg Leu Leu Asn Gly Phe Gly Ala Gly Leu Ala Val
               100                 105                 110
   Thr Leu Val Pro Val Tyr Ile Ser Glu Thr Ala Pro Pro Glu Ile Arg
           115                 120                 125
   Gly Gln Leu Asn Thr Leu Pro Gln Phe Leu Gly Ser Gly Gly Met Phe
       130                 135                 140
   Leu Ser Tyr Cys Met Val Phe Thr Met Ser Leu Ser Asp Ser Pro Ser
   145                 150                 155                 160
   Trp Arg Ala Met Leu Gly Val Leu Ser Ile Pro Ser Leu Leu Tyr Leu
                   165                 170                 175
   Phe Leu Thr Val Phe Tyr Leu Pro Glu Ser Pro Arg Trp Leu Val Ser
               180                 185                 190
   Lys Gly Arg Met Asp Glu Ala Lys Arg Val Leu Gln Gln Leu Cys Gly
           195                 200                 205
   Arg Glu Asp Val Thr Asp Glu Met Ala Leu Leu Val Glu Gly Leu Asp
       210                 215                 220
   Ile Gly Gly Glu Lys Thr Met Glu Asp Leu Leu Val Thr Leu Glu Asp
   225                 230                 235                 240
   His Glu Gly Asp Asp Thr Leu Glu Thr Val Asp Glu Asp Gly Gln Met
                   245                 250                 255
   Arg Leu Tyr Gly Thr His Glu Asn Gln Ser Tyr Leu Ala Arg Pro Val
               260                 265                 270
   Pro Glu Gln Asn Ser Ser Leu Gly Leu Arg Ser Arg His Gly Ser Leu
           275                 280                 285
   Ala Asn Gln Ser Met Ile Leu Lys Asp Pro Leu Val Asn Leu Phe Gly
       290                 295                 300
   Ser Leu His Glu Lys Met Pro Glu Ala Gly Gly Asn Thr Arg Ser Gly
   305                 310                 315                 320
   Ile Phe Pro His Phe Gly Ser Met Phe Ser Thr Thr Ala Asp Ala Pro
                   325                 330                 335
   His Gly Lys Pro Ala His Trp Glu Lys Asp Ile Glu Ser His Tyr Asn
               340                 345                 350
   Lys Asp Asn Asp Asp Tyr Ala Thr Asp Asp Gly Ala Gly Asp Asp Asp
           355                 360                 365
   Asp Ser Asp Asn Asp Leu Arg Ser Pro Leu Met Ser Arg Gln Thr Thr
       370                 375                 380
   Ser Met Asp Lys Asp Met Ile Pro His Pro Thr Ser Gly Ser Thr Leu
   385                 390                 395                 400
   Ser Met Arg Arg His Ser Thr Leu Met Gln Gly Asn Gly Glu Ser Ser
                   405                 410                 415
   Met Gly Ile Gly Gly Gly Trp His Met Gly Tyr Arg Tyr Glu Asn Asp
               420                 425                 430
   Glu Tyr Lys Arg Tyr Tyr Leu Lys Glu Asp Gly Ala Glu Ser Arg Arg
           435                 440                 445
   Gly Ser Ile Ile Ser Ile Pro Gly Gly Pro Asp Gly Gly Gly Ser Tyr
       450                 455                 460
   Ile His Ala Ser Ala Leu Val Ser Arg Ser Val Leu Gly Pro Lys Ser
   465                 470                 475                 480
   Val His Gly Ser Ala Met Val Pro Pro Glu Lys Ile Ala Ala Ser Gly
                   485                 490                 495
   Pro Leu Trp Ser Ala Leu Leu Glu Pro Gly Val Lys Arg Ala Leu Val
               500                 505                 510
   Val Gly Val Gly Ile Gln Ile Leu Gln Gln Phe Ser Gly Ile Asn Gly
           515                 520                 525
   Val Leu Tyr Tyr Thr Pro Gln Ile Leu Glu Arg Ala Gly Val Asp Ile
       530                 535                 540
   Leu Leu Ser Ser Leu Gly Leu Ser Ser Ile Ser Ala Ser Phe Leu Ile
   545                 550                 555                 560
   Ser Gly Leu Thr Thr Leu Leu Met Leu Pro Ala Ile Val Val Ala Met
                   565                 570                 575
   Arg Leu Met Asp Val Ser Gly Arg Arg Ser Leu Leu Leu Trp Thr Ile
               580                 585                 590
   Pro Val Leu Ile Val Ser Leu Val Val Leu Val Ile Ser Glu Leu Ile
           595                 600                 605
   His Ile Ser Lys Val Val Asn Ala Ala Leu Ser Thr Gly Cys Val Val
       610                 615                 620
   Leu Tyr Phe Cys Phe Phe Val Met Gly Tyr Gly Pro Ile Pro Asn Ile
   625                 630                 635                 640
   Leu Cys Ser Glu Ile Phe Pro Thr Arg Val Arg Gly Leu Cys Ile Ala
                   645                 650                 655
   Ile Cys Ala Met Val Phe Trp Ile Gly Asp Ile Ile Val Thr Tyr Ser
               660                 665                 670
   Leu Pro Val Leu Leu Ser Ser Ile Gly Leu Val Gly Val Phe Ser Ile
   675 680 685
   Tyr Ala Ala Val Cys Val Ile Ser Trp Ile Phe Val Tyr Met Lys Val
   690 695 700
   Pro Glu Thr Lys Gly Met Pro Leu Glu Val Ile Thr Asp Tyr Phe Ala
   705 710 715 720
   Phe Gly Ala Gln Ala Gln Ala Ser Ala Pro Ser Lys Asp Ile
   725 730
<210> 2
   <211> 2205
   <212> DNA
   <213> Arabidopsis thaliana
<400> 2
   atgaagggag cgactctcgt tgctctcgcc gccacaatcg gcaatttctt acaaggatgg     60
   gacaatgcca ccattgctgg agctatggtt tatatcaaca aagacttgaa tctaccaacc    120
   tctgttcaag gtcttgtcgt tgctatgtca ttgatcggtg caacggtcat cacgacttgc    180
   tcaggaccga tatctgattg gctcggcaga cgccccatgc tcattttatc atcagttatg    240
   tatttcgtct gcggtttgat aatgttgtgg tctcccaatg tctatgttct gtgctttgct    300
   aggcttctta atgggtttgg tgccgggctc gcggttacac ttgtccctgt ttacatttct    360
   gaaaccgctc ctccggagat cagaggacag ttaaatactc tccctcagtt tcttggctct    420
   ggtggaatgt ttttgtcata ctgtatggtt ttcactatgt ccctgagtga ctcccctagc    480
   tggagagcca tgctcggtgt cctctcgatc ccttctcttc tttatttgtt tctcacggtg    540
   ttttatttgc ccgagtctcc tcgttggctg gttagtaaag gaagaatgga cgaggctaag    600
   cgagttcttc aacagttatg tggcagagaa gatgttaccg atgagatggc tttactagtt    660
   gaaggactag atataggagg agaaaaaaca atggaagatc tcttagtaac tttggaggat    720
   catgaaggtg atgatacact tgaaaccgtt gatgaggatg gacaaatgcg gctttatgga    780
   acccacgaga atcaatcgta ccttgctaga cctgtcccag aacaaaatag ctcacttggg    840
   ctacgctctc gccacggaag cttagcaaac caaagcatga tccttaaaga tccgctcgtc    900
   aatctttttg gcagtctcca cgagaagatg ccagaagcag gcggaaacac tcggagtggg    960
   attttccctc atttcggaag catgttcagt actactgccg atgcgcctca cggtaaaccg   1020
   gctcattggg aaaaggacat agagagccat tacaacaaag acaatgatga ctatgcgact   1080
   gatgatggtg cggaacaaaa acttatctcg gcagaagatt tgcgtagccc cttaatgtcg   1140
   cgccagacca caagcatgga caaggatatg atcccacatc ctacaagtgg aagcacttta   1200
   agcatgagac gacacagtac gcttatgcaa ggcaacggcg aaagtagcat gggaattggt   1260
   ggtggttggc atatgggata tagatacgaa aacgatgaat acaagaggta ttatcttaaa   1320
   gaagatggag ctgaatctcg ccgtggctcg atcatctcta ttcccggagg tccggatggt 1380
   ggaggcagct acattcacgc ttctgccctt gtaagcagat ctgttcttgg tcctaaatca 1440
   gttcatggat ccgccatggt tcccccggag aaaattgctg cctctggacc actctggtct 1500
   gctcttcttg aacctggtgt taagcgtgcc ttggttgttg gtgtcggcat tcaaatactg 1560
   cagcagtttt caggtatcaa tggagttctc tactacactc ctcagattct cgaacgggct 1620
   ggcgtagata ttcttctttc gagcctcgga ctaagttcca tctctgcgtc attcctcatc 1680
   agcggtttaa caacattact catgctccca gccattgtcg ttgccatgag actcatggat 1740
   gtatccggaa gaaggtcatt acttctctgg acaatcccag ttctcattgt ctcacttgtc 1800
   gtccttgtca tcagcgagct catccacatc agcaaagtcg tgaacgcagc actctccaca 1860
   ggttgtgtcg tgctctactt ctgcttcttc gtgatgggtt acggtcccat tccaaacatc 1920
   ctctgttctg aaatcttccc aacaagagtc cgtggtctct gcatcgccat atgtgctatg 1980
   gtcttttgga ttggagacat tattgtcacg tactcacttc ccgttctcct cagctcgatc 2040
   ggactagttg gtgttttcag catttacgct gcggtttgcg ttatctcatg gatcttcgtt 2100
   tacatgaaag tcccggagac taaaggcatg cctttggaag ttatcacaga ctactttgcc 2160
   tttggagctc aagctcaagc ttctgctcct tctaaggata tataa 2205
<210> 3
   <211> 5166
   <212> DNA
   <213> Arabidopsis thaliana
<400> 3
   ctaaattgta agcgttaata ttttgttaaa attcgcgtta aatttttgtt aaatcagctc      60
   attttttaac caataggccg aaatcggcaa aatcccttat aaatcaaaag aatagaccga      120
   gatagggttg agtgttgttc cagtttggaa caagagtcca ctattaaaga acgtggactc     180
   caacgtcaaa gggcgaaaaa ccgtctatca gggcgatggc ccactacgtg aaccatcacc     240
   ctaatcaagt tttttggggt cgaggtgccg taaagcacta aatcggaacc ctaaagggag     300
   cccccgattt agagcttgac ggggaaagcc ggcgaacgtg gcgagaaagg aagggaagaa     360
   agcgaaagga gcgggcgcta gggcgctggc aagtgtagcg gtcacgctgc gcgtaaccac     420
   cacacccgcc gcgcttaatg cgccgctaca gggcgcgtcc cattcgccat tcaggctgcg     480
   caactgttgg gaagggcgat cggtgcgggc ctcttcgcta ttacgccagc tggcgaaagg     540
   gggatgtgct gcaaggcgat taagttgggt aacgccaggg ttttcccagt cacgacgttg     600
   taaaacgacg gccagtgagc gcgcgtaata cgactcacta tagggcgaat tgggtaccgg     660
   gccccccctc gaggtcgacg gtatcgataa gcttgattta tatatcctta gaaggagcag     720
   aagcttgagc ttgagctcca aaggcaaagt agtctgtgat aacttccaaa ggcatgcctt     780
   tagtctccgg gactttcatg taaacgaaga tccatgagat aacgcaaacc gcagcgtaaa    840
   tgctgaaaac tccaactagt tcgatcgagc tgaggagaac gggaagtgag tacgtgacaa    900
   taatgtctcc aatccaaaag accatagcac atatggcgat gcagagacca cggtctgctt    960
   gttgggaaga tttcagaaca gaggatgttt ggaatggacc gtaacccatc acgaagaagc   1020
   agaagtagag cacgacacaa cctgtggaga gtgctgcgtt cacgactttg ctgatgtgga   1080
   tgagctcgct gatgacaagg acgacaagtg agacaatgag aactgggatt gtccagagaa   1140
   gtaatgacct tcttccggat acatccatga gtctcatggc aacgacaatg gctgggagca   1200
   tgagtaatgt tgttaaaccg ctgatgagga atgacgcaga gatggaactt agtccgaggc   1260
   tcgaaagaag aatatctacg ccagcccgtt cgagaatctg aggagtgtag tagagaactc   1320
   cattgatacc tgaaaactgc tgcagtattt gaatgccgac accaacaacc aaggcacgct   1380
   taacaccagg ttcaagaaga gcagaccaga gtggtccaga ggcagcaatt ttctccgggg   1440
   gaaccatggc ggatccatga actgatttag gaccaagaac agatctgctt acaagggcag   1500
   aagcgtgaat gtagctgcct ccaccatccg gacctccggg aatagagatg atcgagccac   1560
   ggcgagattc agctccatct tctttaagat aatacctctt gtattcatcg ttttcgtatc   1620
   tatatcccat atgccaacca ccaccaattc ccatgctact ttcgccgttg ccttgcataa   1680
   gcgtactgtg tcgtctcatg cttaaagtgc ttccacttgt aggatgtggg atcatatcct   1740
   tgtccatgct tgtggtctgg cgcgacatta aggggctacg caaatcttct tccgagataa   1800
   gtttttgttc cgcaccatca tcagtcgcat agtcatcatt gtctttgttg taatggctct   1860
   ctatgtcctt ttcccaatga gccggtttac cgtgaggcgc atcggcagta gtactgaaca   1920
   tgcttccgaa atgagggaaa atcccactcc gagtgtttcc gcctgcttct ggcatcttct   1980
   cgtggagact gccaaaaaga ttgacgagcg gatctttaag gatcatgctt tggtttgcta   2040
   agcttccgtg gcgagagcgt agcccaagtg agctattttg ttctgggaca ggtctagcaa   2100
   ggtacgattg attctcgtgg gttccataaa gccgtatttg tccatcctca tcaacggttt   2160
   caagtgtatc atcaccttca tgatcctcca aagttactaa gagatcttcc attgtttttt   2220
   ctcctcctat atctagtcct tcaactagta aagccatctc atcggtaaca tcttctctgc   2280
   cacataactg ttgaagaact cgcttagcct cgtccattct tcctttacta accagccaac   2340
   gaggagactc gggcaaataa aacaccgtga gaaacaaata aagaagagaa gggatcgaga   2400
   ggacaccgag catggctctc cagctagggg agtcactcag ggacatagtg aaaaccatac   2460
   agtatgacaa aaacattcca ccagagccaa gaaactgagg gagagtattt aactgtcctc   2520
   tgatctccgg aggagcggtt tcagaaatgt aaacagggac aagtgtaacc gcgagcccgg   2580
   caccaaaccc attaagaagc ctagcaaagc acagaacata gacattggga gaccacaaca   2640
   ttatcaaacc gcagacgaaa tacataactg atgataaaat gagcatgggg cgtctgccga 2700
   gccaatcaga tatcggtcct gagcaagtcg tgatgaccgt tgcaccgatc aatgacatag 2760
   caacgacaag accttgaaca gaggttggta gattcaagtc tttgttgata taaaccatag 2820
   ctccagcaat ggtggcattg tcccatcctt gtaagaaatt gccgattgtg gcggcgagag 2880
   caacgagagt cgctcccttc atatcgaatt cctgcagccc gggggatcca ctagttctag 2940
   agcggccgcc accgcggtgg agctccagct tttgttccct ttagtgaggg ttaattgcgc 3000
   gcttggcgta atcatggtca tagctgtttc ctgtgtgaaa ttgttatccg ctcacaattc 3060
   cacacaacat acgagccgga agcataaagt gtaaagcctg gggtgcctaa tgagtgagct 3120
   aactcacatt aattgcgttg cgctcactgc ccgctttcca gtcgggaaac ctgtcgtgcc 3180
   agctgcatta atgaatcggc caacgcgcgg ggagaggcgg tttgcgtatt gggcgctctt 3240
   ccgcttcctc gctcactgac tcgctgcgct cggtcgttcg gctgcggcga gcggtatcag 3300
   ctcactcaaa ggcggtaata cggttatcca cagaatcagg ggataacgca ggaaagaaca 3360
   tgtgagcaaa aggccagcaa aaggccagga accgtaaaaa ggccgcgttg ctggcgtttt 3420
   tccataggct ccgcccccct gacgagcatc acaaaaatcg acgctcaagt cagaggtggc 3480
   gaaacccgac aggactataa agataccagg cgtttccccc tggaagctcc ctcgtgcgct 3540
   ctcctgttcc gaccctgccg cttaccggat acctgtccgc ctttctccct tcgggaagcg 3600
   tggcgctttc tcatagctca cgctgtaggt atctcagttc ggtgtaggtc gttcgctcca 3660
   agctgggctg tgtgcacgaa ccccccgttc agcccgaccg ctgcgcctta tccggtaact 3720
   atcgtcttga gtccaacccg gtaagacacg acttatcgcc actggcagca gccactggta 3780
   acaggattag cagagcgagg tatgtaggcg gtgctacaga gttcttgaag tggtggccta 3840
   actacggcta cactagaagg acagtatttg gtatctgcgc tctgctgaag ccagttacct 3900
   tcggaaaaag agttggtagc tcttgatccg gcaaacaaac caccgctggt agcggtggtt 3960
   tttttgtttg caagcagcag attacgcgca gaaaaaaagg atctcaagaa gatcctttga 4020
   tcttttctac ggggtctgac gctcagtgga acgaaaactc acgttaaggg attttggtca 4080
   tgagattatc aaaaaggatc ttcacctaga tccttttaaa ttaaaaatga agttttaaat 4140
   caatctaaag tatatatgag taaacttggt ctgacagtta ccaatgctta atcagtgagg 4200
   cacctatctc agcgatctgt ctatttcgtt catccatagt tgcctgactc cccgtcgtgt 4260
   agataactac gatacgggag ggcttaccat ctggccccag tgctgcaatg ataccgcgag 4320
   acccacgctc accggctcca gatttatcag caataaacca gccagccgga agggccgagc 4380
   gcagaagtgg tcctgcaact ttatccgcct ccatccagtc tattaattgt tgccgggaag 4440
   ctagagtaag tagttcgcca gttaatagtt tgcgcaacgt tgttgccatt gctacaggca 4500
   tcgtggtgtc acgctcgtcg tttggtatgg cttcattcag ctccggttcc caacgatcaa 4560
   ggcgagttac atgatccccc atgttgtgca aaaaagcggt tagctccttc ggtcctccga   4620
   tcgttgtcag aagtaagttg gccgcagtgt tatcactcat ggttatggca gcactgcata   4680
   attctcttac tgtcatgcca tccgtaagat gcttttctgt gactggtgag tactcaacca   4740
   agtcattctg agaatagtgt atgcggcgac cgagttgctc ttgcccggcg tcaatacggg   4800
   ataataccgc gccacatagc agaactttaa aagtgctcat cattggaaaa cgttcttcgg   4860
   ggcgaaaact ctcaaggatc ttaccgctgt tgagatccag ttcgatgtaa cccactcgtg   4920
   cacccaactg atcttcagca tcttttactt tcaccagcgt ttctgggtga gcaaaaacag   4980
   gaaggcaaaa tgccgcaaaa aagggaataa gggcgacacg gaaatgttga atactcatac   5040
   tcttcctttt tcaatattat tgaagcattt atcagggtta ttgtctcatg agcggataca   5100
   tatttgaatg tatttagaaa aataaacaaa taggggttcc gcgcacattt ccccgaaaag   5160
   tgccac                                                              5166
<210> 4
   <211> 7242
   <212> DNA
   <213> Arabidopsis thaliana
<400> 4
   tgaccaagtc agcttggcac tggccgtcgt tttacaacgt cgtgactggg aaaaccctgg     60
   cgttacccaa cttaatcgcc ttgcagcaca tccccctttc gccagctggc gtaatagcga    120
   agaggcccgc accgatcgcc cttcccaaca gttgcgcagc ctgaatggcg aatgggaaat    180
   tgtaaacgtt aatattttgt taatattttg ttaaaattcg cgttaaattt ttgttaaatc    240
   agctcatttt ttaaccaata ggccgaaatc ggcaaaatcc cttataaatc aaaagaatag    300
   accgagatag ggttgagtgt tgttccagtt tggaacaaga gtccactatt aaagaacgtg    360
   gactccaacg tcaaagggcg aaaaaccgtc tatcagggcg atggcccact acgtgaacca    420
   tcaccctaat caagtttttt ggggtcgagg tgccgtaaag cactaaatcg gaaccctaaa    480
   gggatgcccc gatttagagc ttgacgggga aagccggcga acgtggcgag aaaggaaggg    540
   aagaaagcga aaggagcggg cgctagggcg ctggcaagtg tagcggtcac gctgcgcgta    600
   accaccacac ccgccgcgct taatgcgccg ctacagggcg cgtcaggtgg cacttttcgg    660
   ggaaatgtgc gcggaacccc tatttgttta tttttctaaa tacattcaaa tatgtatccg    720
   ctcatgagac aataaccctg ataaatgctt caataatatt gaaaaaggaa gagtatgagt    780
   attcaacatt tccgtgtcgc ccttattccc ttttttgcgg cattttgcct tcctgttttt    840
   gctcacccag aaacgctggt gaaagtaaaa gatgctgaag atcagttggg tgcacgagtg    900
   ggttacatcg aactggatct caacagcggt aagatccttg agagttttcg ccccgaagaa    960
   cgttttccaa tgatgagcac ttttaaagtt ctgctatgtg gcgcggtatt atcccgtatt   1020
   gacgccgggc aagagcaact cggtcgccgc atacactatt ctcagaatga cttggttgag 1080
   tactcaccag tcacagaaaa gcatcttacg gatggcatga cagtaagaga attatgcagt 1140
   gctgccataa ccatgagtga taacactgcg gccaacttac ttctgacaac gatcggagga 1200
   ccgaaggagc taaccgcttt tttgcacaac atgggggatc atgtaactcg ccttgatcgt 1260
   tgggaaccgg agctgaatga agccatacca aacgacgagc gtgacaccac gatgcctgta 1320
   gcaatggcaa caacgttgcg caaactatta actggcgaac tacttactct agcttcccgg 1380
   caacaattaa tagactggat ggaggcggat aaagttgcag gaccacttct gcgctcggcc 1440
   cttccggctg gctggtttat tgctgataaa tctggagccg gtgagcgtgg gtctcgcggt 1500
   atcattgcag cactggggcc agatggtaag ccctcccgta tcgtagttat ctacacgacg 1560
   gggagtcagg caactatgga tgaacgaaat agacagatcg ctgagatagg tgcctcactg 1620
   attaagcatt ggtaactgtc agaccaagtt tactcatata tactttagat tgatttaaaa 1680
   cttcattttt aatttaaaag gatctaggtg aagatccttt ttgataatct catgaccaaa 1740
   atcccttaac gtgagttttc gttccactga gcgtcagacc ccgtagaaaa gatcaaagga 1800
   tcttcttgag atcctttttt tctgcgcgta atctgctgct tgcaaacaaa aaaaccaccg 1860
   ctaccagcgg tggtttgttt gccggatcaa gagctaccaa ctctttttcc gaaggtaact 1920
   ggcttcagca gagcgcagat accaaatact gtccttctag tgtagccgta gttaggccac 1980
   cacttcaaga actctgtagc accgcctaca tacctcgctc tgctaatcct gttaccagtg 2040
   gctgctgcca gtggcgataa gtcgtgtctt accgggttgg actcaagacg atagttaccg 2100
   gataaggcgc agcggtcggg ctgaacgggg ggttcgtgca cacagcccag cttggagcga 2160
   acgacctaca ccgaactgag atacctacag cgtgagctat gagaaagcgc cacgcttccc 2220
   gaagggagaa aggcggacag gtatccggta agcggcaggg tcggaacagg agagcgcacg 2280
   agggagcttc cagggggaaa cgcctggtat ctttatagtc ctgtcgggtt tcgccacctc 2340
   tgacttgagc gtcgattttt gtgatgctcg tcaggggggc ggagcctatg gaaaaacgcc 2400
   agcaacgcgg cctttttacg gttcctggcc ttttgctggc cttttgctca catgttcttt 2460
   cctgcgttat cccctgattc tgtggataac cgtattaccg cctttgagtg agctgatacc 2520
   gctcgccgca gccgaacgac cgagcgcagc gagtcagtga gcgaggaagc ggaagagcgc 2580
   ccaatacgca aaccgcctct ccccgcgcgt tggccgattc attaatgcag ctggcacgac 2640
   aggtttcccg actggaaagc gggcagtgag cgcaacgcaa ttaatgtgag ttagctcact 2700
   cattaggcac cccaggcttt acactttatg cttccggctc gtatgttgtg tggaattgtg 2760
   agcggataac aatttcacac aggaaacagc tatgaccatg attacgaatt tggccaagtc 2820
   ggcctctaat acgactcact atagggagct cgtcgagcgg ccgctcgacg aattaattcc 2880
   aatcccacaa aaatctgagc ttaacagcac agttgctcct ctcagagcag aatcgggtat 2940
   tcaacaccct catatcaact actacgttgt gtataacggt ccacatgccg gtatatacga 3000
   tgactggggt tgtacaaagg cggcaacaaa cggcgttccc ggagttgcac acaagaaatt 3060
   tgccactatt acagaggcaa gagcagcagc tgacgcgtac acaacaagtc agcaaacaga 3120
   caggttgaac ttcatcccca aaggagaagc tcaactcaag cccaagagct ttgctaaggc 3180
   cctaacaagc ccaccaaagc aaaaagccca ctggctcacg ctaggaacca aaaggcccag 3240
   cagtgatcca gccccaaaag agatctcctt tgccccggag attacaatgg acgatttcct 3300
   ctatctttac gatctaggaa ggaagttcga aggtgaaggt gacgacacta tgttcaccac 3360
   tgataatgag aaggttagcc tcttcaattt cagaaagaat gctgacccac agatggttag 3420
   agaggcctac gcagcaggtc tcatcaagac gatctacccg agtaacaatc tccaggagat 3480
   caaatacctt cccaagaagg ttaaagatgc agtcaaaaga ttcaggacta attgcatcaa 3540
   gaacacagag aaagacatat ttctcaagat cagaagtact attccagtat ggacgattca 3600
   aggcttgctt cataaaccaa ggcaagtaat agagattgga gtctctaaaa aggtagttcc 3660
   tactgaatct aaggccatgc atggagtcta agattcaaat cgaggatcta acagaactcg 3720
   ccgtgaagac tggcgaacag ttcatacaga gtcttttacg actcaatgac aagaagaaaa 3780
   tcttcgtcaa catggtggag cacgacactc tggtctactc caaaaatgtc aaagatacag 3840
   tctcagaaga ccaaagggct attgagactt ttcaacaaag gataatttcg ggaaacctcc 3900
   tcggattcca ttgcccagct atctgtcact tcatcgaaag gacagtagaa aaggaaggtg 3960
   gctcctacaa atgccatcat tgcgataaag gaaaggctat cattcaagat ctctctgccg 4020
   acagtggtcc caaagatgga cccccaccca cgaggagcat cgtggaaaaa gaagacgttc 4080
   caaccacgtc ttcaaagcaa gtggattgat gtgacatctc cactgacgta agggatgacg 4140
   cacaatccca ctatccttcg caagaccctt cctctatata aggaagttca tttcatttgg 4200
   agaggacacg ctcgaggaat tcgatatgaa gggagcgact ctcgttgctc tcgccgccac 4260
   aatcggcaat ttcttacaag gatgggacaa tgccaccatt gctggagcta tggtttatat 4320
   caacaaagac ttgaatctac caacctctgt tcaaggtctt gtcgttgcta tgtcattgat 4380
   cggtgcaacg gtcatcacga cttgctcagg accgatatct gattggctcg gcagacgccc 4440
   catgctcatt ttatcatcag ttatgtattt cgtctgcggt ttgataatgt tgtggtctcc 4500
   caatgtctat gttctgtgct ttgctaggct tcttaatggg tttggtgccg ggctcgcggt 4560
   tacacttgtc cctgtttaca tttctgaaac cgctcctccg gagatcagag gacagttaaa 4620
   tactctccct cagtttcttg gctctggtgg aatgtttttg tcatactgta tggttttcac 4680
   tatgtccctg agtgactccc ctagctggag agccatgctc ggtgtcctct cgatcccttc 4740
   tcttctttat ttgtttctca cggtgtttta tttgcccgag tctcctcgtt ggctggttag 4800
   taaaggaaga atggacgagg ctaagcgagt tcttcaacag ttatgtggca gagaagatgt 4860
   taccgatgag atggctttac tagttgaagg actagatata ggaggagaaa aaacaatgga 4920
   agatctctta gtaactttgg aggatcatga aggtgatgat acacttgaaa ccgttgatga 4980
   ggatggacaa atacggcttt atggaaccca cgagaatcaa tcgtaccttg ctagacctgt 5040
   cccagaacaa aatagctcac ttgggctacg ctctcgccac ggaagcttag caaaccaaag 5100
   catgatcctt aaagatccgc tcgtcaatct ttttggcagt ctccacgaga agatgccaga 5160
   agcaggcgga aacactcgga gtgggatttt ccctcatttc ggaagcatgt tcagtactac 5220
   tgccgatgcg cctcacggta aaccggctca ttgggaaaag gacatagaga gccattacaa 5280
   caaagacaat gatgactatg cgactgatga tggtgcggaa caaaaactta tctcggaaga 5340
   agatttgcgt agccccttaa tgtcgcgcca gaccacaagc atggacaagg atatgatccc 5400
   acatcctaca agtggaagca ctttaagcat gagacgacac agtacgctta tgcaaggcaa 5460
   cggcgaaagt agcatgggaa ttggtggtgg ttggcatatg ggatatagat acgaaaacga 5520
   tgaatacaag aggtattatc ttaaagaaga tggagctgaa tctcgccgtg gctcgatcat 5580
   ctctattccc ggaggtccgg atggtggagg cagctacatt cacgcttctg cccttgtaag 5640
   cagatctgtt cttggtccta aatcagttca tggatccgcc atggttcccc cggagaaaat 5700
   tgctgcctct ggaccactct ggtctgctct tcttgaacct ggtgttaagc gtgccttggt 5760
   tgttggtgtc ggcattcaaa tactgcagca gttttcaggt atcaatggag ttctctacta 5820
   cactcctcag attctcgaac gggctggcgt agatattctt ctttcgagcc tcggactaag 5880
   ttccatctct gcgtcattcc tcatcagcgg tttaacaaca ttactcatgc tcccagccat 5940
   tgtcgttgcc atgagactca tggatgtatc cggaagaagg tcattacttc tctggacaat 6000
   cccagttctc attgtctcac ttgtcgtcct tgtcatcagc gagctcatcc acatcagcaa 6060
   agtcgtgaac gcagcactct ccacaggttg tgtcgtgctc tacttctgct tcttcgtgat 6120
   gggttacggt ccattccaaa catcctctgt tctgaaatct tcccaacaag cagaccgtgg 6180
   tctctgcatc gccatatgtg ctatggtctt ttggattgga gacattattg tcacgtactc 6240
   acttcccgtt ctcctcagct cgatcgaact agttggagtt ttcagcattt acgctgcggt 6300
   ttgcgttatc tcatggatct tcgtttacat gaaagtcccg gagactaaag gcatgccttt 6360
   ggaagttatc acagactact ttgcctttgg agctcaagct caagcttctg ctccttctaa 6420
   ggatatataa atcaagctta tcgataagct tggatcctct agagtcctgc tttaatgaga 6480
   tatgcgagac gcctatgatc gcatgatatt tgctttcaat tctgttgtgc acgttgtaaa 6540
   aaacctgagc atgtgtagct cagatcctta ccgccggttt cggttcattc taatgaatat 6600
   atcacccgtt actatcgtat ttttatgaat aatattctcc gttcaattta ctgattgtac 6660
   cctactactt atatgtacaa tattaaaatg aaaacaatat attgtgctga ataggtttat   6720
   agcgacatct atgatagagc gccacaataa caaacaattg cgttttatta ttacaaatcc   6780
   aattttaaaa aaagcggcag aaccggtcaa acctaaaaga ctgattacat aaatcttatt   6840
   caaatttcaa aaggccccag gggctagtat ctacgacaca ccgagcggcg aactaataac   6900
   gttcactgaa gggaactccg gttccccgcc ggcgcgcatg ggtgagattc cttgaagttg   6960
   agtattggcc gtccgctcta ccgaaagtta cgggcaccat tcaacccggt ccagcacggc   7020
   ggccgggtaa ccgacttgct gccccgagaa ttatgcagca tttttttggt gtatgtgggc   7080
   cccaaatgaa gtgcaggtca aaccttgaca gtgacgacaa atcgttgggc gggtccaggg   7140
   cgaattttgc gacaacatgt cgaggctcag caggacctgc aggcatgcaa gctagcttac   7200
   tagtgatgca tattctatag tgtcacctaa atctgcggcc gc                      7242